# EUROPEAN PATENT SPECIFICATION

(11) **EP 3 979 965 B1**
(45) Date of publication and mention of the grant of the patent: **30.07.2025**
(21) Application number: 20742206.4
(22) Date of filing: 13.07.2020
(51) Int. Cl.: A61F 13/08, A61F 5/01, A61F 13/10

(54) **A MEDICAL COMPRESSION GARMENT WITH A SPECIAL JOINT SECTION FOR INCREASED RANGE OF MOTION**
MEDIZINISCHES KOMPRESSIONSKLEIDUNGSSTÜCK MIT EINEM SPEZIELLEN GELENKABSCHNITT FÜR ERHÖHTEN BEWEGUNGSBEREICH
VÊTEMENT DE COMPRESSION MÉDICAL DOTÉ D'UNE SECTION D'ARTICULATION SPÉCIALE POUR UNE PLAGE DE MOUVEMENT ACCRUE

(30) Priority: 19.08.2019 CH 10392019
(43) Date of publication of application: 13.04.2022
(73) Proprietor: Sigvaris AG, 9014 St. Gallen (CH)
(72) Inventor: GANZONI, Levin Andreas, 8052 Zürich (CH)
(74) Representative: E. Blum & Co. AG
(86) International application number: PCT/EP2020/069750
(87) International publication number: WO 2021/032361

(56) References cited:
- WO-A1-95/16416
- WO-A1-95/16416
- WO-A2-2009/004282
- WO-A2-2009/004282
- KR-A- 19990 083 740
- KR-A- 19990 083 740
- US-A- 4 653 492
- US-A- 4 653 492

## Description

### Technical Field

The present invention relates to a medical compression garment for applying compression to a patient's body part comprising a first material adapted to be wrapped around the body part and a second material fixated on a section of the first material. Furthermore, the present invention relates to a method for producing such a medical compression garment.

### Background Art

Medical compression garments are used to provide a pressure or a compressive force, respectively, to a human body part, especially to a limb such as an arm or a leg for e.g. the treatment of venous diseases and lymphatic disorders.

Medical compression garments limit the range of motion of the human extremities, especially the joint movement since many medical compression garments comprise almost inelastic materials.

WO 2009/004282 shows a roll of dressing which can be applied to the skin. The dressing comprises a stretchable fabric outer layer and an inner layer of hydrophilic polyurethane plastics film. The film is bonded to the outer layer by means of a suitable adhesive Upon curing of the adhesive, the tension in the outer layer is released. This release causes the outer layer and the film to contract.

US 4,653,492 discloses an elastic bandage have a resilient elastic layer and a relatively non-resilient layer to limit the stretching of the elastic layer.

KR19990083740 discloses a disposable band which can easily be attached to a knee or an elbow as well as a flat wound part.

### Disclosure of the Invention

The object of the present invention is to provide a medical compression garment that restricts the range of motion of compressed body parts as little as possible.

In order to meet this object, a first aspect of the invention introduces a medical compression garment according to claim 1.

The medical compression garment for applying compression to a patient's body part comprises
- a first material adapted to be wrapped around the body part, and
- a second material fixated on a section of the first material. The second material collapses the first material such that the first material forms wrinkles along the section.

This has the advantage that the first material forms wrinkles in a controlled manner. Uncontrolled wrinkles, which are uncomfortable, are avoided. If the wrinkles are located at a joint, e.g. at an elbow, the range of motion of the joint and the wearing comfort are improved.

The wrinkles are arranged at an elbow part of the medical compression garment. The elbow part of a medical compression garment is the part which is shaped for positioning on the human elbow. Furthermore, the first material comprises at least one closing part for fixating the wrapped first material around the body part.

Advantageously, the second material is more elastic than the first material, in particular wherein the stretchability of the second material is twice as high, in particular five times as high, as the stretchability of the first material.

The high stretchability of the second material allows the forming of the wrinkles and ensures a good fit around the joint.

In a preferred embodiment, the stretchability of the first material is increased by the wrinkles in the direction perpendicular to the wrinkles. This improves the wearing comfort and makes the donning of the garment very easy.

Advantageously, the second material extends in longitudinal direction such that the first material forms at least three, in particular at least five, in particular at least eight, in particular at least ten, wrinkles. Only a sufficiently large number of wrinkles allows comfortable wearing at the joint.

In particular, the second material is attached to the first material by gluing, welding, or sewing, in particular between every wrinkle. This results in a robust compression garment, which can be worn for a long time.

In a preferred embodiment, the second material extends over a fraction of the width or the full width of the first material.

In particular, the first material is a textile or leather or plastic or laminated foam.

Preferably, the second material is a textile or foil or plastic or laminated foam.

Furthermore, an object of the present invention is to provide a method for producing the inventive medical compression garment.

The method for producing a medical compression garment comprises the following steps:
- fixating the first material in a fixation device in a relaxed state,
- arranging the second material in a stretched state onto the first material in the fixation device,
- attaching, in particular sewing, the first material to the second material,
- removing the fixation device such that the second material relaxes and that the first material forms wrinkles.

### Brief Description of the Drawings

The above and other features and advantages of the present invention will become more apparent to those of ordinary skill in the art by describing in detail preferred embodiments thereof with reference to the attached drawings in which:
Fig. 1 illustrates an example embodiment of the disclosed medical compression garment applied at an arm;
Fig. 2 is a sectional view of the medical compression garment in a relaxed state showing the wrinkles of the first material;
Fig. 3 shows a medical compression garment in an unwrapped state; and
Fig. 4a, 4b and 4c show the method of producing the disclosed medical compression garment.

### Modes for Carrying Out the Invention

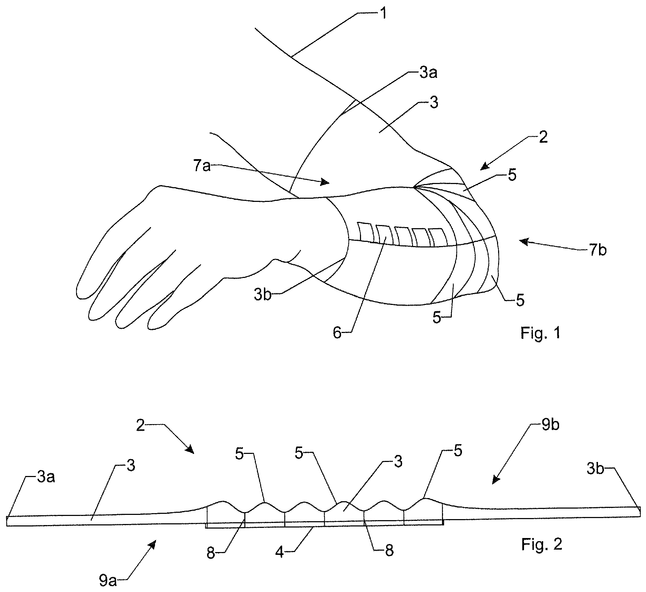

Fig. 1 shows a human arm 1 with an elbow. A medical compression garment 2 is applied to the arm. The medical compression garment comprises a first material 3 which extends from a first end 3a to a second end 3b. The first material 3 extends over the full length of the medical compression garment 2. A second material 4 is fixated in a middle section of the medical compression garment 2. The second material 4 is not visible in Fig. 1 since it is arranged on the inner side of the medical compression garment, i.e. it touches the surface of the arm 1. But the second material 4 collapses the first material 3 such that the first material forms wrinkles 5 along the section where the second material 4 is fixated. The wrinkles 5 are visible in Fig. 1.

With other words, the wrinkles 5 are present regardless of whether the arm is straight or bent. When the arm is bent, the wrinkles 5 are already formed and defined such that no new wrinkles are formed on the inner side 7a of the elbow which would press uncomfortably hard on the elbow joint. On the outer side 7b of the elbow, the preformed wrinkles 5 are stretched, such that the medical compression garment lies comfortably on the elbow.

The medical compression garment 2 is a garment which is wrapped around the arm and fixated by closing parts 6.

Fig. 2 shows a sectional view of the medical compression garment 2 along its length axis. The medical compression garment 2 comprises a first material 3, which extends from the first end 3a to the second end 3b, and a second material 4 which is arranged in the middle section of the medical compression garment 2.

The first material 3 and the second material 4 are attached to each other by seams 8 between every wrinkle 5. The second material 4 is more elastic than the first material 3. Since the second material 4 is seamed to the first material 3 in a stretched state, it collapses the first material 3 in a relaxed state and six wrinkles 5 are formed. The section where the second material 4 is attached to the first material 3 is called the joint part or the elbow part of the medical compression garment, since this part is intended to be placed at the human elbow.

In the present embodiment, the inner side 9a of the medical compression garment, where the second material 4 is attached, is intended to touch the skin of the human. The outer side 9b lies on the opposite side of the inner side 9a. It does not touch the skin of the human body and is adapted such that the closing parts 6 can be attached to the outer side.

In the present embodiment, the first material 3 is a textile, in particular a spacer fabric, and the second material 4 is also a textile, but it is more elastic than the textile of the first material 3.

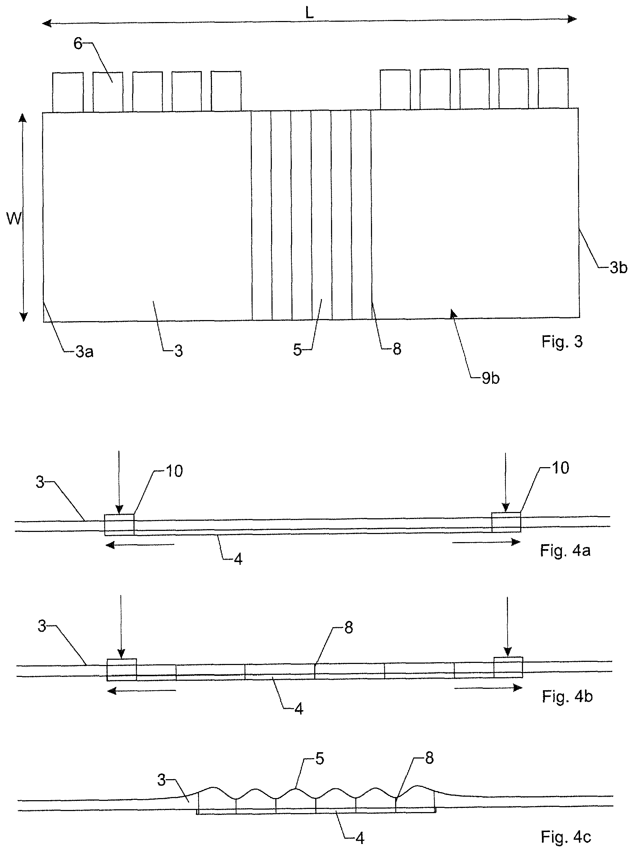

Fig. 3 shows the medical compression garment from a top view in an unwrapped state. The first material 3 extends over the full length L and the second material 4 (not shown in Fig. 3) is attached to the first material 3 such that wrinkles 5 are formed over the full width W in the middle part of the medical compression garment 2. The medical compression garment 2 is wrapped around the body part and fixated by the closing parts 6.

Furthermore, the second material 4 can be attached to the first material 3 such that the stretchability of the first material 3 is increased in the direction perpendicular to the wrinkles 5, i.e. perpendicular to the direction of the width W.

The figures 4a, 4b and 4c illustrate the method for producing the medical compression garment. The method comprises the following steps:
- the first material 3 is fixated in a fixation device 10 in a relaxed state (Fig. 4a);
- the second material 4 is arranged in a stretched state onto the first material 3 and fixated in the fixation device 10 (Fig. 4a);
- the first material 3 is attached to the second material 4 by sewing the first material 3 and the second material 4 together (Fig. 4b);
- the fixation device 10 is removed such that the second material 4 relaxes and that the first material 3 forms wrinkles 5.

The present invention has so far been described for some specific embodiments shown in the fig-ures. It must be noted that it also can be practiced differently. The scope of the invention is defined by the claims.

## Claims

1. A medical compression garment for applying compression to a patient's arm comprising
- a first material (3) adapted to be wrapped around the arm,
- a second material (4) fixated on a section of the first material (3), wherein the second material (4) collapses the first material (3) such that the first material (3) forms wrinkles (5) along the section,
**characterized in that**
the wrinkles (5) are arranged at an elbow part of the medical compression garment, and the first material (3) comprises at least one closing part (6) for fixating the wrapped first material (3) around the body part.

2. The medical compression garment of claim 1, wherein the second material (4) is more elastic than the first material (3), in particular wherein the stretchability of the second material (4) is twice as high, in particular five times as high, as the stretchability of the first material (3)

3. The medical compression garment according to any of the preceding claims, wherein the stretchability of the first material (3) is increased by the wrinkles (5) in the direction perpendicular to the wrinkles (5).

4. The medical compression garment according to any of the preceding claims, wherein the second material (4) extends in longitudinal direction such that the first material (3) forms at least three, in particular at least five, in particular at least eight, in particular at least ten, wrinkles (5).

5. The medical compression garment according to any of the preceding claims, wherein the second material (4) is attached to the first material by gluing, welding, or sewing, in particular between every wrinkle (5).

6. The medical compression garment according to any of the preceding claims, wherein the second material (4) extends over a fraction of the width (W) or the full width (W) of the first material (3).

7. The medical compression garment according to any of the preceding claims, wherein the first material (3) is a textile or leather or plastic or laminated foam.

8. The medical compression garment according to any of the preceding claims, wherein the second material (4) is a textile or foil or plastic or laminated foam.

9. A method for producing a medical compression garment according to one of the preceding claims with the following steps:
- fixating the first material (3) in a fixation device (10) in a relaxed state,
- arranging the second material (4) in a stretched state onto the first material (3) in the fixation device (10),
- attaching the first material (3) to the second material (4),
- removing the fixation device (10) such that the second material (4) relaxes and that the first material (3) forms wrinkles (5).

## Patentansprüche

1. Medizinisches Kompressionskleidungsstück zum Ausüben von Kompression auf einen Arm eines Patienten, umfassend
- ein erstes Material (3), ausgestaltet um es um den Arm zu wickeln,
- ein zweites Material (4), das auf einem Abschnitt des ersten Materials (3) fixiert ist, wobei das zweite Material (4) das erste Material (3) zusammenfallen lässt, so dass das erste Material (3) entlang des Abschnitts Falten (5) bildet,
**dadurch gekennzeichnet, dass**
die Falten (5) an einem Ellbogenteil des medizinischen Kompressionsbekleidungsstücks angeordnet sind, und das erste Material (3) mindestens ein Verschlussteil (6) zum Fixieren des eingewickelten ersten Materials (3) um das Körperteil herum umfasst.

2. Medizinisches Kompressionskleidungsstück nach Anspruch 1, wobei das zweite Material (4) elastischer ist als das erste Material (3), insbesondere wobei die Dehnbarkeit des zweiten Materials (4) doppelt so hoch, insbesondere fünfmal so hoch ist wie die Dehnbarkeit des ersten Materials (3).

3. Medizinisches Kompressionskleidungsstück nach einem der vorangehenden Ansprüche, wobei die Dehnbarkeit des ersten Materials (3) durch die Falten (5) in der Richtung senkrecht zu den Falten (5) erhöht wird.

4. Medizinisches Kompressionskleidungsstück nach einem der vorangehenden Ansprüche, wobei sich das zweite Material (4) in Längsrichtung derart erstreckt, dass das erste Material (3) mindestens drei, insbesondere mindestens fünf, insbesondere mindestens acht, insbesondere mindestens zehn Falten (5) bildet.

5. Medizinisches Kompressionskleidungsstück nach einem der vorangehenden Ansprüche, wobei das zweite Material (4) durch Kleben, Schweissen oder Nähen, insbesondere zwischen jeder Falte (5), mit dem ersten Material verbunden ist.

6. Medizinisches Kompressionskleidungsstück nach einem der vorangehenden Ansprüche, wobei sich das zweite Material (4) über einen Bruchteil der Breite (W) oder die volle Breite (W) des ersten Materials (3) erstreckt.

7. Medizinisches Kompressionskleidungsstück nach einem der vorangehenden Ansprüche, wobei das erste Material (3) ein Textil oder Leder oder Kunststoff oder laminierter Schaumstoff ist.

8. Medizinisches Kompressionskleidungsstück nach einem der vorangehenden Ansprüche, wobei das zweite Material (4) ein Textil oder eine Folie oder ein Kunststoff oder laminierter Schaumstoff ist.

9. Verfahren zur Herstellung eines medizinischen Kompressionsbekleidungsstücks nach einem der vorangehenden Ansprüche mit den folgenden Schritten:
- Fixieren des ersten Materials (3) in einer Fixiervorrichtung (10) in einem entspannten Zustand,
- Anordnen des zweiten Materials (4) in einem gestreckten Zustand auf dem ersten Material (3) in der Fixiervorrichtung (10),
- Anbringen des ersten Materials (3) an das zweite Material (4),
- Entfernen der Fixierungsvorrichtung (10), so dass sich das zweite Material (4) entspannt und das erste Material (3) Falten (5) bildet.

## Revendications

1. Vêtement médical de compression pour appliquer une compression au bras d'un patient, comprenant
- un premier matériau (3), adapté à envelopper le bras,
- un second matériau (4) fixé sur une section du premier matériau (3), dans lequel le second matériau (4) contraint le premier matériau (3) de sorte que le premier matériau (3) forme des plis (5) le long de la section,
**caractérisé en ce que**
les plis (5) sont disposés au niveau d'une partie du coude du vêtement médical de compression, et le premier matériau (3) comprend au moins une partie de fermeture (6) pour fixer le premier matériau (3) enveloppant la partie de corps.

2. Le vêtement médical de compression de la revendication 1, dans lequel le second matériau (4) est plus élastique que le premier matériau (3), en particulier dans lequel l'extensibilité du second matériau (4) est deux fois plus élevée, en particulier cinq fois plus élevée, que l'extensibilité du premier matériau (3).

3. Le vêtement médical de compression selon l'une des revendications précédentes, dans lequel l'extensibilité du premier matériau (3) est augmentée par les plis (5) dans la direction perpendiculaire aux plis (5).

4. Le vêtement médical de compression selon l'une des revendications précédentes, dans lequel le second matériau (4) s'étend en direction longitudinale de manière à ce que le premier matériau (3) forme au moins trois, en particulier au moins cinq, en particulier au moins huit, en particulier au moins dix, plis (5).

5. Le vêtement médical de compression selon l'une quelconque des revendications précédentes, dans lequel le second matériau (4) est fixé au premier matériau par collage, soudage ou couture, en particulier entre chaque pli (5).

6. Vêtement médical de compression selon l'une des revendications précédentes, dans lequel le second matériau (4) s'étend sur une fraction de la largeur (W) ou sur toute la largeur (W) du premier matériau (3).

7. Le vêtement médical de compression selon l'une des revendications précédentes, dans lequel le premier matériau (3) est un textile, du cuir, du plastique ou de la mousse stratifiée.

8. Le vêtement médical de compression selon l'une des revendications précédentes, dans lequel le second matériau (4) est un textile, une feuille, un plastique ou une mousse stratifiée.

9. Procédé de fabrication d'un vêtement de compression médicale selon l'une des revendications précédentes, comportant les étapes suivantes :
- fixer le premier matériau (3) à l'état détendu dans un dispositif de fixation (10),
- disposer le second matériau (4) à l'état étiré sur le premier matériau (3) dans le dispositif de fixation (10),
- fixer le premier matériau (3) au second matériau (4),
- retirer le dispositif de fixation (10) de manière à ce que le second matériau (4) se détende et que le premier matériau (3) forme des plis (5).
